# EUROPEAN PATENT SPECIFICATION

(11) **EP 3 923 895 B1**
(45) Date of publication and mention of the grant of the patent: **06.09.2023**
(21) Application number: 20833732.9
(22) Date of filing: 10.12.2020
(51) Int. Cl.: A61H 7/00, A61H 15/00, A61N 1/32

(54) **BODY MASSAGE DEVICE**
KÖRPERMASSAGEVORRICHTUNG
DISPOSITIF DE MASSAGE CORPOREL

(30) Priority: 27.04.2020 ES 202030355
(43) Date of publication of application: 22.12.2021
(73) Proprietor: Termosalud, S.L., 33213 Gijon - Asturias (ES)
(72) Inventor: DEL VALLE FERNÁNDEZ, Iván, 33213 Gijón - Asturias (ES)
(74) Representative: Clarke, Modet y Cía., S.L.
(86) International application number: PCT/EP2020/085519
(87) International publication number: WO 2021/219238

(56) References cited:
- CN-U- 205 586 230
- KR-A- 20200 033 720
- US-A1- 2016 128 605
- US-A1- 2017 319 429

## Description

### FIELD OF THE INVENTION

The present invention falls within the devices used for massaging patients, especially those that have an additional energy source for improving the effect of said massages, and incorporating the monitoring of the effective depth of the treatment.

### BACKGROUND OF THE INVENTION

There are numerous massage devices, which provide the user with the ability to apply a massage homogeneously and without causing fatigue in the therapist.

Some massage devices that use brushes, gears, and drive chains generate excessive friction which negatively affects the effectiveness of the massage.

Other devices comprise a handpiece, that is, a support to be used with the therapist's hand that has a series of spherical elements (also called heads) which press the patient's body when the massage is performed. This handpiece ends in a massage arm which comprises the spherical elements, which can rotate while moving along the patient's body, improving the massage effect. Sometimes, there are sensors for the applied vertical force.

Recently, massage devices have appeared that use a heat supply for accompanying massage movements. This heat source is usually in the form of infrared radiation or Joule currents. Due to this heat, the effect of the massage is improved, since a better preparation of the connective tissue is achieved.

CN 205 586 230 U discloses a device according to the preamble of claim 1.

### SUMMARY OF THE INVENTION

The invention is set out in the appended set of claims.

### SUMMARY OF THE DISCLOSURE

Despite the aforementioned advances, there is room for improvement in existing devices. Existing devices omit important parameters for treatment, even those that combine rotary massage and electromagnetic radiation.

The movable heads apply a rotary massage with much less effort from the therapist, even keeping the handpiece of the massage device static. The present invention applies rotary massage and makes use of the effective depth parameter, which is understood as the distance that it is convenient to reach with the massage.

Determining the effective depth is not trivial. Although there are solutions capable of detecting the vertical force applied in a treatment, this information alone does not enable the effective depth to be identified. The effective depth varies according to the features of the patient (for example, large or thin) and also the specific area of the body. The layer of fat under the skin is the main factor in effective depth, but not the only one.

It has been found that the effective depth is further conditioned by factors such as the elasticity of the skin, hydration or friction of the head on the area to be applied.

In short, based on his subjective experience the therapist must judge whether the massage is performed at appropriate depth and/or pressure.

To address the identified limitations, the present invention is proposed, which discloses a device for body massage which monitors the effectiveness of the treatment by means of the technical features of the main claim.

The massage device has a handpiece comprising a plurality of movable heads for applying force on a patient's body and a massage arm connected to the movable heads. It includes a motor mechanically coupled to the movable heads for conveying motion thereto. It includes measurement means comprising a vertical force sensor for measuring the force applied perpendicularly on the patient's body, and a torque sensor for measuring the resistance to rotation of the movable heads on the patient's body. Optionally, it can incorporate a thermometer for taking the temperature of the area of the patient's body where the heads are applied. It includes an electromagnetic radiation generator for generating electromagnetic radiation with a fixed frequency and intensity, and applying it to the patient's body through the movable heads. It includes a processing unit which, with the information from the measurement means, estimates the effective depth of the treatment on the patient's body. The processing unit determines in real time if, for the estimated effective depth, the applied force is appropriate.

Unless defined otherwise, all terms (both scientific and technical) used in this document are to be interpreted as a person skilled in the art would. It will be understood, therefore, that the terms of common use must be interpreted in the way that a connoisseur of the subject matter would, and not in an idealised or strictly formal way.

Throughout the text, the word "comprises" (and the derivatives thereof, such as "comprising") should not be understood in an exclusive way, but should be understood in the sense that they admit the possibility that what is defined may include additional elements or steps.

### DESCRIPTION OF THE FIGURES

Below is a brief description of each of the figures used to complete the description of the invention that follows. Said figures are related to embodiments of the invention, which are presented as non-limiting examples thereof.
FIG. 1 shows a general view of a massage device according to the invention.
FIG. 2 comprises a cross-sectional view of the handpiece shown in the previous figure, for a better understanding of the operation thereof.
FIG. 3 is a block diagram of the invention.
FIG. 4 illustrates a graph that is used for relating variables.

### Numerical references used in figures

- 1: Massage arm
- 2: Movable heads
- 3: Conductor cable
- 4: Gearmotor
- 5: Electrical insulator
- 6: Static element of drive assembly (ring)
- 7a: Bearing of the dynamic element of the drive assembly
- 7b: Coupling piece of the dynamic element of the drive assembly
- 8: Temperature sensor
- 9: Fastening magnet
- 10: Handpiece
- 11: Power source
- 12: Electromagnetic radiation generator unit
- 13: Processing unit
- 14: Torque sensor
- 15: Vertical force sensor
- 16: Interface
- 17: Indicator
- 18: Control panel

### DETAILED DESCRIPTION OF THE INVENTION

Different aspects of an exemplary embodiment of the present invention are now described, for merely illustrative and non-limiting purposes.

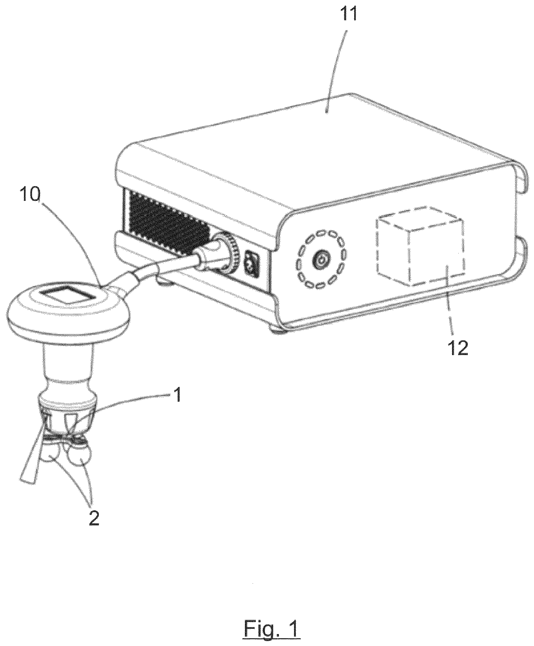

**FIG. 1** shows a general view of a massage device, including a power source 11, comprising an electromagnetic wave generator unit 12, a handpiece 10, which is connected to the power source 11.

This handpiece 10 serves as a support so that the therapist can perform a massage on a patient. The handle 10 in turn comprises various elements that contribute to the purpose of the invention.

In this general figure, a massage arm 1 with a series of massage applicator heads 2 is observed. These heads 2 are intended for pressing the patient's body, for which they comprise a spherical surface, such that contact is favoured.

In addition to pressing, the heads **2** receive energy in electromagnetic radiation waves that is directly radiated onto the patient's body.

The fact that said electromagnetic radiation is directly emitted through the movable heads **2,** instead of through a static support, has a very advantageous effect on the treatment, achieving a therapeutic synergy that enhances the results of each effect separately.

On the one hand, the mechanical activation of different cells and tissues is enhanced, and on the other, peripheral and lymphatic flow is improved due to the application of heat exactly on the area where mechanical activation is taking place.

At the same time, the electrical effect of the passage of electromagnetic waves through the skin causes a biochemical stimulation which favours the elimination of fats and inhibits the accumulation thereof. In addition, it stimulates the cells that produce elastic fibres.

In particular embodiments, the source of electromagnetic waves is modulated with a low-frequency modulating signal (10Hz to 40KHz). This modulation can be done by means of the pulse width control.

Physiologically, the modulation of the electromagnetic wave carrier signal generates an increase in the permeability of plasma cell membranes. On the one hand, the correct dissemination of substances between the extracellular spaces and the cells themselves is favoured; and on the other hand, the exchange capacity of the lymphatic capillaries is improved.

These two effects facilitate the processes of uptake of nutrients and oxygen and the processes of excretion of waste substances to the extracellular environment. An increase in cell permeability in the lymphatic capillaries facilitates a more effective removal of excreted metabolites to the interstitial compartment, reducing inflammation and the consequences thereof.

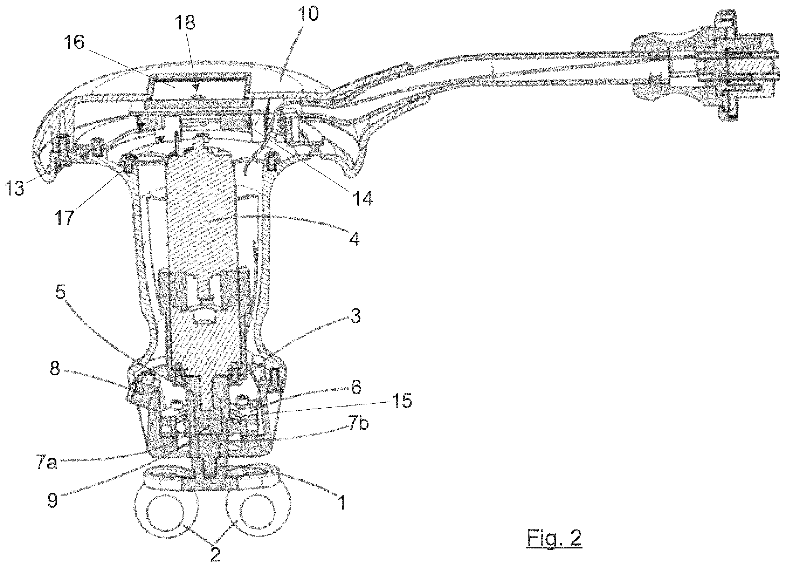

**FIG. 2** comprises a cross-sectional view of one particular embodiment of the handpiece 10 shown in the previous figure, for a better understanding of the mechanical and electrical operation thereof.

Firstly, the mechanical connection that enables the rotation of the movable heads 2 is analysed. The handpiece 10 comprises a motor, electric gearmotor 4, which is electrically powered to the machine. This electric gearmotor 4 has an output shaft covered by an electrical insulator 5, which in turn fits into a dynamic drive element. The purpose of this dynamic drive element is to transmit the motion of the electric gearmotor 4 to the massage arm 1, and is formed by a bearing 7a and the coupling piece for coupling to the massage arm 7b. This bearing ensures the stable rotation of the massage arm 1, by supporting it on a static element 6, which in this case is a ring fastened to the lower portion of the handpiece 10.

Second, the electrical connection between the elements will be analysed. The power source 11 provides energy to an electromagnetic wave generator 12, capable of suitably modulating the carrier signal, and said wave is transmitted along a conductor cable 3. Advantageously, the electromagnetic wave generator can be housed in a machine separate from the handpiece 10. The conductor cable 3 runs through the connection between the power source 11, the electromagnetic wave generator 12 and the handpiece 10 until it makes electrical contact with a static element, the ring 6. Therefore, the conductor cable 3 is free from rotational movements, since it has run through the static portion of the actuator element until it makes contact with the ring 6. Thereby, the electrical connection between the electromagnetic wave generator 12 of the power source 11 and the ring 6 of the handpiece 10 is achieved.

Both the ring 6 and the bearing 7a and the coupling piece of the massage actuator elements 7b are made of conductive material. In addition, the movable heads 2 are also made of conductive material, so that the electromagnetic waves are transmitted to the massage applicators 2 themselves. The massage arm 1 containing the movable heads 2 is fixed to the coupling piece 7b by means of a magnet 9.

The torque sensor 14 measures the resistance to rotation, in one embodiment it is performed with a current sensor incorporated in the electronic control board, which also operates as a processing unit 13. By means of an algorithm described below it is computed combining it with the vertical force sensor 15, to continuously monitor the effective depth in a user interface for the interaction of the therapist.

The processing unit 13, in addition to applying the precise control logic, enables the interaction with the therapist through the interface 16, which contains a control panel 18 for receiving the work command and monitor the parameters of the session. It includes an indicator 17, which by means of a beep informs the therapist of errors or warnings that require his attention during treatment.

Finally, the handpiece 10 further comprises a temperature sensor 8, which continuously measures the evolution of the temperature of the treated area of the patient, being reflected on the interface 16 to be consulted by the therapist. This temperature must be comprised within an established range, generally between 42 °C and 45 °C to facilitate the correct mobilisation of fat. Temperature monitoring enables the therapist to control that the treatment is being appropriately and safely performed. A temperature higher than 45 °C can cause burns. A temperature lower than 42 °C has no effect on body fat.

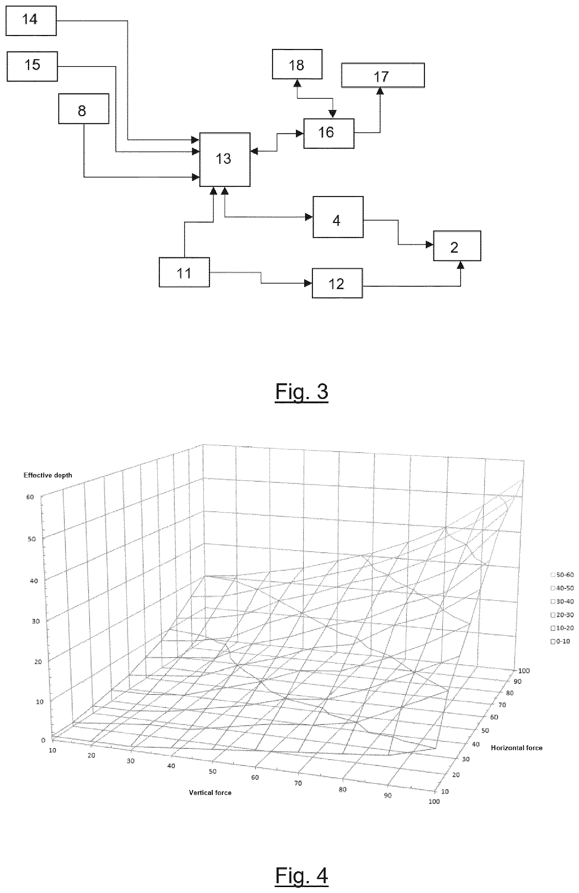

**FIG. 3** schematically illustrates a block diagram of a massage device to show the functional relationships between different elements. A motor or gearmotor 4 is shown that applies rotational motion on the movable heads 2. A set of sensors that includes the temperature sensor 8, the vertical force sensor 15 (vertical force exerted by the therapist) and the torque sensor 14. These sensors perform measurements in order to know the vertical force and torque that the movable heads 2 convey on the patient. These measurements are provided to the processing unit 13.

Moreover, the electromagnetic radiation generator 12 generates electromagnetic radiation with a frequency and intensity previously selected by the therapist according to the type of massage, for example, with the interface 16. The characteristics of the generated radiation are provided to the processing unit 13. This radiation reaches the patient through the movable heads 2. The processing unit 13 analyses the information and establishes whether the massage is being appropriately applied to the patient.

For this purpose, the processing unit 13 calculates the aforementioned parameter called effective depth which informs about the depth of the treatment. This parameter has a value that is mainly a function of the vertical force applied, the torque and the amount of fat. It also depends secondarily on other features of the patient, such as hydration and firmness of the skin.

The processing unit 13 is programmed to control whether the vertical force applied by the therapist is sufficient to reach the fatty layer of the patient. A shallower depth means that the target area where the fat is located is not effectively reached, so the fat liquefies with the electromagnetic radiation but is not properly mobilised. A greater depth means that it reaches the muscle area, which is not appropriate, it is painful and even dangerous.

Given the factors on which they depend, the effective depth varies greatly between patients, so the simple measure of vertical force applied is not enough to determine if the target area is reached.

For this reason, to estimate this effective depth, in one of the embodiments a developed algorithm is implemented that relates different variables taking the values empirically. To obtain the relationship between the two measured parameters, an experienced therapist applies the equipment to a patient and determines when he is in a suitable effective depth range and with what vertical force and resistance to rotation measurements, obtaining the following table (column: vertical force, rows: torque, result: effective depth in mm):

| | Horizontal force (%) | | | | | | | | | |
|---|---|---|---|---|---|---|---|---|---|---|
| Vertical force (%) | 10 | 20 | 30 | 40 | 50 | 60 | 70 | 80 | 90 | 100 |
| 10 | 1 | 1 | 1 | 2 | 3 | 4 | 5 | 6 | 7 | 10 |
| 20 | 1 | 2 | 3 | 3 | 5 | 7 | 8 | 10 | 15 | 20 |
| 30 | 2 | 2 | 3 | 5 | 7 | 10 | 11 | 15 | 20 | 25 |
| 40 | 3 | 3 | 4 | 7 | 10 | 14 | 15 | 19 | 24 | 30 |
| 50 | 4 | 5 | 6 | 10 | 13 | 17 | 19 | 25 | 29 | 36 |
| 60 | 6 | 7 | 8 | 13 | 16 | 20 | 23 | 28 | 32 | 41 |
| 70 | 8 | 9 | 11 | 16 | 20 | 24 | 27 | 31 | 36 | 47 |
| 80 | 12 | 13 | 15 | 20 | 23 | 27 | 30 | 35 | 40 | 50 |
| 90 | 16 | 17 | 20 | 23 | 26 | 29 | 33 | 38 | 44 | 52 |
| 100 | 20 | 21 | 23 | 25 | 28 | 31 | 35 | 40 | 47 | 55 |

This table can be supplemented with further considerations, for example characteristics of the patient such as age, sex, etc. to achieve greater selectivity.

By determining the effective depth, the incorporation of a skinfold calliper for measuring body fat is prevented, reducing the possibility of error in the process, as it is automatically generated by the system without depending on the therapist.

**FIG. 4** illustrates a graph which serves to relate the vertical force, torque and effective depth variables, corresponding to the values in the table. Note that the torque and vertical force values are dimensionless, since they correspond to percentages of the direct value of the sensor implemented in the equipment from which the table was empirically taken.

Usually, the embodiments of the invention enable the radiation to be applied to be selected based on the type of patient and/or treatment. During the application of the massage, the torque is monitored to ensure it is appropriate. Otherwise, in some embodiments, a warning signal is issued to the therapist.

Additionally, types of patients can be defined according to their body mass: large, normal, thin. Additionally, body areas can be defined where the treatment is to be performed. Certain areas have a greater or lesser tendency to accumulate fat.

The above are examples for easily customising the treatment.

In other embodiments, the above empirical relationship can be implemented by means of equivalent formulas which associate vertical force and torque values with depth.

In the embodiments discussed, the therapist is the one who decides to apply more or less force assisted by the massage device. In other embodiments, the rotation force can be regulated to control the torque and thus maintain the effective depth of the treatment independent of the force applied by the therapist, albeit within a reasonable working range, giving a warning if it is not outside the appropriate range. In other, more automated embodiments, vertical force control may be implemented, although it would complicate the mechanical system.

## Claims

1. A body massage device comprising:
a handpiece (10) comprising a plurality of movable heads (2) for applying force on a patient's body and a massage arm (1) connected to the movable heads (2);
a motor (4) mechanically coupled to the plurality of movable heads (2) for conveying motion thereto;
measurement means comprising a vertical force sensor (15) configured for measuring the force applied perpendicularly on the patient's body;
an electromagnetic radiation generator (12) configured for generating electromagnetic radiation with a fixed frequency and intensity, wherein the movable heads (2) are electrically connected to the electromagnetic radiation generator (12) for receiving electromagnetic radiation and applying it on the patient's body;
a processing unit (13) configured for receiving information from the measurement means;
**characterised in that** the massage device further comprises a torque sensor (14) configured for measuring the resistance to rotation of the movable heads (2) on the patient's body;
wherein the processing unit (13) is configured for estimating the effective depth of the treatment on the patient's body, wherein, for estimating the effective depth of the treatment, the processing unit (13) comprises a register that stores at least vertical force and torque values and relates them to an effective depth value for the patient and for determining in real time, if, for the effective depth, the applied vertical force is appropriate.

2. The massage device according to claim 1, wherein the measurement means further comprise a temperature sensor (8), so that the processing unit (13) determines whether the temperature is appropriate when it is within a predetermined range.

3. The massage device according to claim 1 or 2, further comprising an indicator (17) for warning if the vertical force, torque and/or temperature is appropriate.

4. The massage device according to claim 3, wherein the processing unit (13) is configured to act on the motor (4) if the vertical force or torque is not appropriate.

5. The massage device according to any one of the preceding claims 2 to 4, wherein the processing unit (13) is configured for acting on the electromagnetic radiation generator (12) if the temperature is not appropriate.

6. The massage device according to any one of the preceding claims, further comprising an interface (16) for setting values at least for the torque, frequency and intensity of the electromagnetic radiation.

7. The massage device according to any one of the preceding claims, wherein the electromagnetic radiation generated is a wave modulated at a frequency value comprised between 10 Hz and 40 KHz.

8. The massage device according to claim 7, wherein the modulation is by pulse width.

9. The massage device according to any one of the preceding claims, wherein the heads (2) are spherical in shape.

10. The massage device according to any one of the preceding claims, further comprising a power supply (12) configured for supplying energy to the electromagnetic radiation generation means (12) and to the motor (4).

11. The massage device according to claim 10, wherein the power supply (12) is housed separately from the handpiece (10).

## Patentansprüche

1. Körpermassagevorrichtung, umfassend:
ein Handstück (10), das eine Vielzahl von beweglichen Köpfen (2) zum Anwenden von Kraft auf den Körper eines Patienten umfasst, und einen Massagearm (1), der mit den beweglichen Köpfen (2) verbunden ist;
einen Motor (4), der mechanisch mit der Vielzahl von beweglichen Köpfen (2) gekoppelt ist, um die Bewegung dorthin zu fördern;
Messmittel, die einen vertikalen Kraftsensor (15) umfassen, der zum Messen der senkrecht auf den Körper des Patienten angewandten Kraft ausgebildet ist;
einen elektromagnetischen Strahlungsgenerator (12), der so ausgebildet ist, dass er elektromagnetische Strahlung mit einer festgelegten Frequenz und Intensität erzeugt, wobei die beweglichen Köpfe (2) elektrisch mit dem elektromagnetischen Strahlungsgenerator (12) verbunden sind, um elektromagnetische Strahlung zu empfangen und auf den Körper des Patienten anzuwenden;
eine Verarbeitungseinheit (13), die zum Empfangen von Informationen von den Messmitteln ausgebildet ist;
**dadurch gekennzeichnet, dass** die Massagevorrichtung ferner einen Drehmomentsensor (14) umfasst, der so ausgebildet ist, dass er den Widerstand gegen die Drehung der beweglichen Köpfe (2) am Körper des Patienten misst;
wobei die Verarbeitungseinheit (13) zum Schätzen der effektiven Tiefe der Behandlung auf dem Körper des Patienten ausgebildet ist, wobei zum Schätzen der effektiven Tiefe der Behandlung die Verarbeitungseinheit (13) ein Register umfasst, das mindestens vertikale Kraft- und Drehmomentwerte speichert und diese mit einem effektiven Tiefenwert für den Patienten in Beziehung setzt und in Echtzeit bestimmt, ob für die effektive Tiefe die angewandte vertikale Kraft angemessen ist.

2. Massagevorrichtung nach Anspruch 1, wobei die Messmittel ferner einen Temperatursensor (8) umfassen, so dass die Verarbeitungseinheit (13) bestimmt, ob die Temperatur angemessen ist, wenn sie sich in einem vorbestimmten Bereich befindet.

3. Massagevorrichtung nach Anspruch 1 oder 2, die ferner eine Anzeige (17) umfasst, die warnt, wenn die vertikale Kraft, das Drehmoment und/oder die Temperatur angemessen sind.

4. Massagevorrichtung nach Anspruch 3, wobei die Verarbeitungseinheit (13) so ausgebildet ist, dass sie auf den Motor (4) wirkt, wenn die vertikale Kraft oder das Drehmoment nicht angemessen ist.

5. Massagevorrichtung nach einem der vorhergehenden Ansprüche 2 bis 4, wobei die Verarbeitungseinheit (13) so ausgebildet ist, dass sie auf den elektromagnetischen Strahlungsgenerator (12) wirkt, wenn die Temperatur nicht angemessen ist.

6. Massagevorrichtung nach einem der vorhergehenden Ansprüche, die ferner eine Schnittstelle (16) zum Einstellen von Werten mindestens für das Drehmoment, die Frequenz und die Intensität der elektromagnetischen Strahlung umfasst.

7. Massagevorrichtung nach einem der vorhergehenden Ansprüche, wobei die erzeugte elektromagnetische Strahlung eine Welle ist, die mit einem Frequenzwert im Bereich zwischen 10 Hz und 40 KHz moduliert ist.

8. Massagevorrichtung nach Anspruch 7, wobei die Modulation durch Pulsbreite erfolgt.

9. Massagevorrichtung nach einem der vorhergehenden Ansprüche, wobei die Köpfe (2) kugelförmig sind.

10. Massagevorrichtung nach einem der vorhergehenden Ansprüche, die ferner eine Stromversorgung (12) umfasst, die so ausgebildet ist, dass sie die Mittel (12) zur Erzeugung elektromagnetischer Strahlung und den Motor (4) mit Energie versorgt.

11. Massagevorrichtung nach Anspruch 10, wobei die Stromversorgung (12) getrennt vom Handstück (10) aufgenommen ist.

## Revendications

1. Dispositif de massage corporel comprenant :
une pièce à main (10) comprenant une pluralité de têtes mobiles (2) pour appliquer une force sur le corps d'un patient et un bras de massage (1) relié aux têtes mobiles (2) ;
un moteur (4) mécaniquement couplé à la pluralité de têtes mobiles (2) pour transmettre un mouvement à celles-ci ;
des moyes de mesure comprenant un capteur de force vertical (15) configuré pour mesurer la force appliquée perpendiculairement sur le corps du patient ;
un générateur de rayonnement électromagnétique (12) configuré pour générer un rayonnement électromagnétique avec une fréquence et une intensité fixes, dans lequel les têtes mobiles (2) sont électriquement reliées au générateur de rayonnement électromagnétique (12) pour recevoir le rayonnement électromagnétique et l'appliquer sur le corps du patient ;
une unité de traitement (13) configurée pour recevoir des informations à partir des moyes de mesure ;
**caractérisé en ce que** le dispositif de massage comprend en outre un capteur de couple (14) configuré pour mesurer la résistance à la rotation des têtes mobiles (2) sur le corps du patient ;
dans lequel l'unité de traitement (13) est configurée pour estimer la profondeur efficace du traitement sur le corps du patient, dans lequel, pour estimer la profondeur efficace du traitement, l'unité de traitement (13) comprend un registre qui stocke au moins des valeurs de couple et de force vertical et les rapporte à une valeur de profondeur efficace pour le patient et pour déterminer, en temps réel, si, pour la profondeur efficace, la force vertical appliquée est appropriée.

2. Dispositif de massage selon la revendication 1, dans lequel les moyes de mesure comprennent en outre un capteur de température (8), de sorte que l'unité de traitement (13) détermine si la température est appropriée lorsqu'elle se trouve dans une plage prédéterminée.

3. Dispositif de massage selon la revendication 1 ou 2, comprenant en outre un indicateur (17) pour avertir si la force vertical, le couple et/ou la température sont appropriés.

4. Dispositif de massage selon la revendication 3, dans lequel l'unité de traitement (13) est configurée pour agir sur le moteur (4) si la force vertical ou le couple ne sont pas appropriés.

5. Dispositif de massage selon l'une quelconque des revendications 2 à 4 précédentes, dans lequel l'unité de traitement (13) est configurée pour agir sur le générateur de rayonnement électromagnétique (12) si la température n'est pas appropriée.

6. Dispositif de massage selon l'une quelconque des revendications précédentes, comprenant en outre une interface (16) pour établir des valeurs au moins pour le couple, la fréquence et l'intensité du rayonnement électromagnétique.

7. Dispositif de massage selon l'une quelconque des revendications précédentes, dans lequel le rayonnement électromagnétique généré onde modulée à une valeur de fréquence comprise entre 10 Hz et 40 KHz.

8. Dispositif de massage selon la revendication 7, dans lequel la modulation est par largeur d'impulsion.

9. Dispositif de massage selon l'une quelconque des revendications précédentes, dans lequel les têtes (2) sont sous forme sphérique.

10. Dispositif de massage selon l'une quelconque des revendications précédentes, comprenant en outre une source de puissance (12) configurée pour fournir de l'énergie aux moyens de génération de rayonnement électromagnétique (12) et au moteur (4).

11. Dispositif de massage selon la revendication 10, dans lequel la source de puissance (12) est logée séparée de la pièce à main (10).
